(19) Europäisches Patentamt
European Patent Office
Office européen des brevets

(11) Veröffentlichungsnummer: **0 542 038 A1**

(12) **EUROPÄISCHE PATENTANMELDUNG**

(21) Anmeldenummer: **92118400.8**

(51) Int. Cl.[5]: **C07C 329/02**

(22) Anmeldetag: **28.10.92**

(30) Priorität: **11.11.91 DE 4137012**

(43) Veröffentlichungstag der Anmeldung:
**19.05.93 Patentblatt 93/20**

(84) Benannte Vertragsstaaten:
**BE CH DE ES FR GB IT LI NL**

(71) Anmelder: **BASF Aktiengesellschaft**
**Carl-Bosch-Strasse 38**
**W-6700 Ludwigshafen(DE)**

(72) Erfinder: **Wettling, Thomas, Dr.**
**Trifelsring 11**
**W-6703 Limburgerhof(DE)**
Erfinder: **Henkelmann, Jochem, Dr.**
**Merianstrasse 5**
**W-6700 Ludwigshafen(DE)**
Erfinder: **Troetsch-Schaller, Irene, Dr.**
**Hannongstrasse 5**
**W-6710 Frankenthal(DE)**

(54) **Verfahren zur Herstellung von Chlorothiolformiaten.**

(57) Verfahren zur Herstellung von Chlorothiolformiaten der allgemeinen Formel I

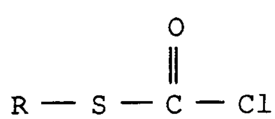

(I),

in der

R $C_3$- bis $C_8$-Cycloalkyl, $C_4$- bis $C_{20}$-Cycloalkyl-alkyl oder gegebenenfalls durch $C_2$- bis $C_4$-Acyl und/oder $C_2$- bis $C_4$-Acyloxy ein- bis dreifach substituiertes $C_1$- bis $C_{30}$-Alkyl, $C_2$- bis $C_{20}$-Alkenyl, $C_2$- bis $C_{20}$-Alkinyl oder gegebenenfalls durch $C_1$- bis $C_4$-Alkyl, $C_1$- bis $C_4$-Alkoxy, Halogen, Nitro, Cyano, $C_2$- bis $C_8$-Dialkylamino, $C_2$- bis $C_4$-Acyl und/oder $C_2$- bis $C_4$-Acyloxy ein- bis dreifach substituiertes Aryl, $C_7$- bis $C_{20}$-Aralkyl, Heterocycloalkyl oder $C_3$- bis $C_{20}$-Heterocycloalkyl-alkyl

bedeutet, indem man Thiole der allgemeinen Formel II

R-SH (II),

in der der Substituent R die oben genannten Bedeutungen hat, mit Phosgen in Gegenwart katalytischer Mengen einer organischen Phosphorverbindung der allgemeinen Formel III

$R^1R^2R^3P(X)_n$ (III),

in der

$R^1,R^2,R^3$ Wasserstoff, $C_1$- bis $C_{20}$-Alkyl, $C_2$- bis $C_{20}$-Alkenyl, Halogen, Aryl oder $C_7$- bis $C_{20}$-Aralkyl bedeutet, wobei $R^1$ und $R^2$, $R^1$ und $R^3$, $R^2$ und $R^3$, $R^5$ und $R^6$ eine gesättigte oder ungesättigte $C_2$- bis $C_5$-Alkylenkette

X Sauerstoff oder Schwefel und

n 0 oder 1

bedeuten, bei Temperaturen von (-20) bis +180 °C und Drücken von 0,01 bis 50 bar umsetzt.

EP 0 542 038 A1

Die vorliegende Erfindung betrifft ein Verfahren zur Herstellung von Chlorothiolformiaten aus Thiolen und Phosgen in Gegenwart katalytischer Mengen einer organischen Phosphorverbindung.

Da diese Reaktion relativ langsam und häufig auch nur unvollständig verläuft, wurde verschiedentlich empfohlen, sie in Gegenwart von Katalysatoren vorzunehmen.

Aus der US-A-3,299,114 ist die Herstellung von Chlorothiolformiaten, die auch als Chlorothiolcarbonate oder S-Ester der Chlorthiokohlensäure bezeichnet werden, durch Phosgenierung der entsprechenden Thiole (Mercaptane) bekannt, wobei als Katalysatoren offenkettige oder heterocyclische tertiäre Amine oder heterocyclische aromatische Stickstoffbasen vom Typ des Pyridins verwendet werden. Die hierbei erzielten Ausbeuten an den Chlorothiolformiaten lassen jedoch zu wünschen übrig, und außerdem geben diese Basen insofern zu verfahrenstechnischen Schwierigkeiten Anlaß, als die aus ihnen bei der Phosgenierung gebildeten Hydrochloride im Reaktionsgemisch unlöslich sind und somit zusätzliche Trennoperationen erforderlich machen.

Der Erfindung lag daher die Aufgabe zugrunde, den zuvor genannten Nachteilen abzuhelfen.

Demgemäß wurde ein neues und verbessertes Verfahren zur Herstellung von Chlorothiolformiaten der allgemeinen Formel I

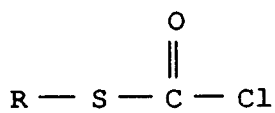 (I),

in der

R $C_3$- bis $C_8$-Cycloalkyl, $C_4$- bis $C_{20}$-Cycloalkyl-alkyl oder gegebenenfalls durch $C_2$- bis $C_4$-Acyl und/oder $C_2$- bis $C_4$-Acyloxy ein- bis dreifach substituiertes $C_1$- bis $C_{30}$-Alkyl, $C_2$- bis $C_{20}$-Alkenyl, $C_2$- bis $C_{20}$-Alkinyl oder gegebenenfalls durch $C_1$- bis $C_4$-Alkyl, $C_1$- bis $C_4$-Alkoxy, Halogen, Nitro, Cyano, $C_2$- bis $C_8$-Dialkylamino, $C_2$- bis $C_4$-Acyl und/oder $C_2$- bis $C_4$-Acyloxy ein- bis dreifach substituiertes Aryl, $C_7$- bis $C_{20}$-Aralkyl, Heterocycloalkyl oder $C_3$- bis $C_{20}$-Heterocycloalkyl-alkyl

bedeutet, gefunden, welches dadurch gekennzeichnet ist, daß man Thiole der allgemeinen Formel II

R-SH (II),

in der der Substituent R die oben genannten Bedeutungen hat, mit Phosgen in Gegenwart katalytischer Mengen einer organischen Phosphorverbindung der allgemeinen Formel III

$R^1R^2R^3P(X)_n$ (III),

in der

$R^1$,$R^2$,$R^3$ Wasserstoff, $C_1$- bis $C_{20}$-Alkyl, $C_6$- bis $C_{14}$-Aryloxy, $C_2$- bis $C_{20}$-Alkenyl, Halogen, Aryl oder $C_7$- bis $C_{20}$-Aralkyl bedeutet, wobei $R^1$ und $R^2$, $R^1$ und $R^3$, $R^2$ und $R^3$, $R^5$ und $R^6$ eine gesättigte oder ungesättigte $C_2$- bis $C_5$-Alkylenkette

X Sauerstoff oder Schwefel und

n 0 oder 1

bedeuten, bei Temperaturen von (-20) bis +180 °C und Drücken von 0,01 bis 50 bar umsetzt.

Das erfindungsgemäße Verfahren läßt sich wie folgt durchführen:

Man kann das Thiol II mit der organischen Phosphorverbindung III vorlegen und anschließend Phosgen ein- bzw. durchleiten.

Das erfindungsgemäße Verfahren kann kontinuierlich oder diskontinuierlich durchgeführt werden. Ein kontinuierliches Verfahren kann beispielsweise im Reaktionsrohr, in einer Rührkesselkaskade, in einem Schlaufenreaktor oder in einer Gegenstromkolonne durchgeführt werden.

Man kann das erfindungsgemäße Verfahren als Flüssigphasenreaktion bei Temperaturen von (-20) bis 180 °C, bevorzugt von 0 bis 120 °C, besonders bevorzugt von 40 bis 100 °C und Drücken von 0,01 bis 50 bar, bevorzugt 0,5 bis 5 bar, besonders bevorzugt bei Normaldruck (Atmosphärendruck), vorzugsweise in homogener flüssiger Phase durchführen. Homogene flüssige Phasen sind beispielsweise die Schmelze der Thiole II oder die Thiole II in einem inerten Lösungsmittel.

Die organische Phosphorverbindung kann in einer Menge von beispielsweise 0,001 bis 20 Mol-%, bevorzugt 0,2 bis 10 Mol-%, besonders bevorzugt 0,5 bis 5 Mol-%, bezogen auf das Thiol II eingesetzt

werden.

Das Molverhältnis von Phosgen zum Thiol II beträgt 1:1 bis 2:1, bevorzugt 1:1 bis 1,5:1, besonders bevorzugt 1:1 bis 1,2:1. Die Verwendung von größeren Überschüssen als 2:1 ist zwar möglich, aber allgemein nicht angezeigt, da sie keine weiteren Vorteile bringt.Die Verwendung von weniger als der stöchiometrischen Menge Phosgen ist möglich, bringt aber im allgemeinen keinen Vorteil, da hierbei nicht umgesetztes Thiol abgetrennt werden muß und die Ausbeute sinkt.

Da die Menge der organischen Phosphorverbindung normalerweise nur sehr gering ist, ist es meistens nicht erforderlich, sie von den Verfahrensprodukten abzutrennen, denn bei den weiteren Reaktionen dieser Zwischenprodukte stören die organischen Phosphorverbindungen in geringen Konzentrationen im allgemeinen nicht.

Selbstverständlich können auch Mischungen von organischen Phosphorverbindungen als Katalysatoren eingesetzt werden.

Die Mitverwendung von Lösungsmitteln ist im allgemeinen nicht erforderlich, es sei denn, das eingesetzte Thiol II und/oder das Verfahrenprodukt I wären unter den Reaktionsbedingungen nicht flüssig. Als Lösungsmittel eignen sich beispielsweise ein aliphatischer oder aromatischer Kohlenwasserstoff, wie Pentan, Hexan, Cyclohexan, Toluol, Xylol oder Benzol, halogenierte Kohlenwasserstoffe, wie Trichlorethan, Methylenchlorid, Chlorbenzol oder Dichlorbenzol oder Ester, wie Ethylacetat oder Butylacetat, Ether wie Dioxan und Tetrahydrofuran und Lactame wie N-Methylpyrrolidon oder das durch Phosgenierung entstehende Chlorformiat des entsprechenden Phenols.Das gleiche gilt für die Aufarbeitung der Reaktionsgemische.

Nach den bisherigen Beobachtungen ist das erfindungsgemäße Verfahren von der Art der Thiole (II) nicht erkennbar abhängig, sieht man von den unterschiedlichen Reaktionsgeschwindigkeiten der Thiole, die auch bei den bisher bekannten Ausführungsformen dieser Reaktion festzustellen waren, ab.

Die Substituenten R, $R^1$, $R^2$, $R^3$ und X in den Verbindungen I, II und III haben folgende Bedeutungen:

R

- $C_3$- bis $C_8$-Cycloalkyl wie Cyclopropyl, Cyclobutyl, Cyclopentyl, Cyclohexyl, Cycloheptyl und Cyclooctyl, bevorzugt Cyclopentyl, Cyclohexyl und Cyclooctyl,
- $C_4$- bis $C_{20}$-Alkyl-cycloalkyl, bevorzugt $C_4$- bis $C_{12}$-Cycloalkyl-alkyl wie Cyclopropyl-methyl, Cyclobutyl-methyl, Cyclopentyl-methyl, Cyclohexyl-methyl, Cyclopropyl-ethyl, Cyclobutyl-ethyl, Cyclopentyl-ethyl, Cyclohexyl-ethyl, Cyclopropyl-propyl, Cyclobutyl-propyl, yclopentyl-propyl und Cyclohexyl-propyl, besonders bevorzugt Cyclopentyl-methyl, Cyclohexyl-methyl, Cyclopentyl-ethyl und Cyclohexyl-ethyl.
- $C_1$- bis $C_{30}$-Alkyl, bevorzugt $C_2$- bis $C_{20}$-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl und iso-Dodecyl, besonders bevorzugt C2- bis C4-Alkyl wie Ethyl, n-Propyl, iso-Propyl, n-Butyl, iso-Butyl, sec.-Butyl und tert.-Butyl,
- $C_2$- bis $C_{20}$-Alkenyl, bevorzugt $C_2$- bis $C_8$-Alkenyl wie Vinyl, Allyl, But-2-en-1-yl, But-4-en-1-yl, But-4-en-2-yl, Pent-2-en-1-yl und 2,2-Dimethylpent-1-en-1-yl, besonders bevorzugt C2-C6-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl und 5-Hexenyl,
- $C_2$-$C_{20}$-Alkinyl wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl und 5-Hexinyl,
- durch $C_2$- bis $C_4$-Acyl und/oder $C_2$- bis $C_4$-Acyloxy ein- bis dreifach substituiertes $C_1$- bis $C_{30}$-Alkyl,
- durch $C_2$- bis $C_4$-Acyl und/oder $C_2$- bis $C_4$-Acyloxy ein- bis dreifach substituiertes $C_2$- bis $C_{20}$-Alkenyl,

- durch $C_2-$ bis $C_4-$Acyl und/oder $C_2-$ bis $C_4-$Acyloxy ein– bis dreifach substituiertes $C_2-$ bis $C_{20}-$Alkinyl,
- Aryl wie Phenyl, 1–Naphthyl, 2–Naphthyl, 9–Naphthyl und Biphenyl, bevorzugt Phenyl,
- $C_7-$ bis $C_{20}-$Aralkyl, bevorzugt $C_7-$ bis $C_{12}-$Aralkyl wie Benzyl, Phenyl–ethyl, 1–Naphthyl–methyl und Biphenylmethyl;
- Heterocycloalkyl, wie ein 5– oder 6–gliedriger Ring mit einem oder zwei O–, N– und/oder S–Atomen im Ring, der aromatisch oder nicht aromatisch sein kann, wie 2– oder 3–Furyl, 2– oder 3–Thienyl, 2– oder 3–Pyrrolyl, 2– oder 4–Imidazolyl, 2– oder 3–Oxazolyl, 2– oder 3–Thiazolyl, Pyridinyl, Morpholyl, Thiomorpholyl und Pyrazolyl,
- durch $C_1-$ bis $C_4-$Alkyl, $C_1-$ bis $C_4-$Alkoxy, Halogen, Nitro, Cyano, $C_2-$ bis $C_8-$Dialkylamino, $C_2-$ bis $C_4-$Acyl und/oder $C_2-$ bis $C_4-$Acyloxy ein– bis dreifach substituiertes Aryl,
- durch $C_1-$ bis $C_4-$Alkyl, $C_1-$ bis $C_4-$Alkoxy, Halogen, Nitro, Cyano, $C_2-$ bis $C_8-$Dialkylamino, $C_2-$ bis $C_4-$Acyl und/oder $C_2-$ bis $C_4-$Acyloxy ein– bis dreifach substituiertes $C_7-$ bis $C_{20}-$Aralkyl,
- durch $C_1-$ bis $C_4-$Alkyl, $C_1-$ bis $C_4-$Alkoxy, Halogen, Nitro, Cyano, $C_2-$ bis $C_8-$Dialkylamino, $C_2-$ bis $C_4-$Acyl und/oder $C_2-$ bis $C_4-$Acyloxy ein– bis dreifach substituiertes Heterocycloalkyl,
- durch $C_1-$ bis $C_4-$Alkyl, $C_1-$ bis $C_4-$Alkoxy, Halogen, Nitro, Cyano, $C_2-$ bis $C_8-$Dialkylamino, $C_2-$ bis $C_4-$Acyl und/oder $C_2-$ bis $C_4-$Acyloxy ein– bis dreifach substituiertes $C_3-$ bis $C_{20}-$Heterocycloalkyl–alkyl,

$R^1$, $R^2$, $R^3$

- unabhängig voneinander
- $C_1-$ bis $C_{20}-$Alkyl, bevorzugt $C_1-$ bis $C_8-$Alkyl wie Methyl, Ethyl, n–Propyl, iso–Propyl, n–Butyl, iso–Butyl, sec.–Butyl, tert.–Butyl, n–Pentyl, iso–Pentyl, sec.–Pentyl, neo–Pentyl, 1,2–Dimethylpropyl, n–Hexyl, iso–Hexyl, sec.–Hexyl, n–Heptyl, iso–Heptyl, n–Octyl, iso–Octyl, besonders bevorzugt $C_4-$ bis $C_8-$Alkyl wie n–Butyl, iso–Butyl, sec.–Butyl und tert.–Butyl, n–Pentyl, n–Hexyl, n–Heptyl und n–Octyl,
- $C_2-$ bis $C_{20}-$Alkenyl, bevorzugt $C_2-$ bis $C_8-$Alkenyl wie Vinyl, Allyl, But–2–en–1–yl, But–4–en–1–yl, But–4–en–2–yl, Pent–2–en–1–yl und 2,2–Dimethylpent–1–en–1–yl,
- Halogen wie Fluor, Chlor, Brom und Jod, bevorzugt Fluor, Chlor und Brom, besonders bevorzugt Chlor und Brom,
- Aryl, wie Phenyl, 1–Naphthyl, 2–Naphthyl, 1–Anthryl, 2–Anthryl und 9–Anthryl, bevorzugt Phenyl, 1–Naphthyl und 2–Naphthyl, besonders bevorzugt Phenyl,
- $C_6-$ bis $C_{14}-$Aryloxy, wie Phenoxy, Tolyloxy, Xylyloxy, Mesityloxy, bevorzugt Phenoxy, Tolyloxy besonders bevorzugt Phenoxy,
- $C_7-$ bis $C_{20}-$Aralkyl, bevorzugt $C_7-$ bis $C_{12}-$Phenylalkyl wie Benzyl, 1–Phenethyl, 2–Phenethyl, 1–Phenyl–propyl, 2–Phenyl–propyl, 3–Phenyl–propyl, 1–Phenyl–butyl, 2–Phenyl–butyl, 3–Phenyl–butyl und 4–Phenyl–butyl, besonders bevorzugt Benzyl, 1–Phenethyl und 2–Phenethyl,
- $R^1$ und $R^2$, $R^1$ und $R^3$, $R^2$ und $R^3$, $R^5$ und $R^6$ eine gesättigte oder ungesättigte $C_2-$ bis $C_5-$Alkylenkette wie $-(CH_2)_2-$, $-(CH_2)_3-$, $-(CH_2)_4-$ und $-(CH_2)_5-$,

X

- Schwefel oder Sauerstoff, bevorzugt Sauerstoff.

n

- 0 oder 1.

Als Beispiele für organische Phosphorverbindungen der allgemeinen Formel III seien genannt:

- Trialkylphosphinoxide mit $C_1-C_{12}-$Alkylgruppen, wie das Tributylphosphinoxid und das Trioctylphosphinoxid,
- Isomerengemische von Hexyl–, Heptyl–, Octylphosphinoxiden wie Cyanex®
- Triarylphosphinoxide wie das Triphenylphosphinoxid,
- Trialkyl– oder Triaryl–phosphine wie das Triphenyl–phosphin,
- Triarylphosphite wie das Triphenylphosphit.

Die Chlorothiolformiate I eignen sich als Zwischenprodukte für die Synthese von organischen Verbindungen, insbesondere von physiologi schen Wirkstoffen wie Arznei– und Pflanzenschutzmitteln.

Beispiele

Beispiele 1 bis 7

In eine Mischung aus 146 g (1 mol) n-Octanthiol (Octylmercaptan) und X mmol einer organischen Phosphorverbindung wurden bei einer Temperatur von T °C im Laufe von t Stunden 110 g (1,1 mol) Phosgen eingeleitet. Anschließend wurde das Gemisch noch 1 h bei der Reaktionstemperatur gehalten.

Danach wurde überschüssiges Phosgen, sowie restlicher Chlorwasserstoff mit Stickstoff aus dem Gemisch ausgeblasen. Das so erhaltene Rohprodukt wurde gaschromatographisch untersucht.

Einzelheiten zu diesen Versuchen sind der nachstehenden Tabelle zu entnehmen.

Tabelle

| Beispiel | Org. Phosphorverb. | X [mmol] | T [°C] | t [h] | GC-Analyse [Fl.-%] Chlorformiat | Carbonat | Thiol | Ausbeute [%] |
|---|---|---|---|---|---|---|---|---|
| 1 | Triphenylphosphit | 2 | 80 | 4 | 98,1 | 0,3 | 0,7 | 97,4 |
| 2 | Triphenylphosphin | 2 | 80 | 2,5 | 94,8 | 0,2 | - | 92,3 |
| 3 | Trioctylphosphinoxid | 2 | 80 | 1 | 98,9 | 0,3 | - | 98,7 |
| 4 | Tributylphosphinoxid | 2 | 80 | 1 | 98,9 | 0,1 | - | 98,6 |
| 5 | Trioctylphosphinoxid | 1 | 80 | 1 | > 99 | 0,1 | - | 99,3 |
| 6 | Trioctylphospinoxid | 0,5 | 80 | 1 | > 99 | 0,1 | - | 99,3 |
| 7 | Cyanex® | 0,5 | 80 | 1 | > 99 | 0,1 | - | 99,2 |

Cyanex®: Ein bei Raumtemperatur flüssiges Isomerengemisch von unterschiedlich substituierten $C_6$- bis $C_{10}$-Alkylphosphinoxiden.

## Patentansprüche

1. Verfahren zur Herstellung von Chlorothiolformiaten der allgemeinen Formel I

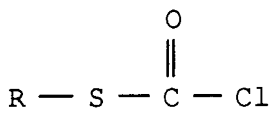 (I),

in der

R $C_3$- bis $C_8$-Cycloalkyl, $C_4$- bis $C_{20}$-Cycloalkyl-alkyl oder gegebenenfalls durch $C_2$- bis $C_4$-Acyl und/oder $C_2$- bis $C_4$-Acyloxy ein- bis dreifach substituiertes $C_1$- bis $C_{30}$-Alkyl, $C_2$- bis $C_{20}$-Alkenyl, $C_2$- bis $C_{20}$-Alkinyl oder gegebenenfalls durch $C_1$- bis $C_4$-Alkyl, $C_1$- bis $C_4$-Alkoxy, Halogen, Nitro, Cyano, $C_2$- bis $C_8$-Dialkylamino, $C_2$- bis $C_4$-Acyl und/oder $C_2$- bis $C_4$-Acyloxy ein- bis dreifach substituiertes Aryl, $C_7$- bis $C_{20}$-Aralkyl, Heterocycloalkyl oder $C_3$- bis $C_{20}$-Heterocycloalkyl-alkyl

bedeutet, dadurch gekennzeichnet, daß man Thiole der allgemeinen Formel II

R-SH (II),

in der der Substituent R die oben genannten Bedeutungen hat, mit Phosgen in Gegenwart katalytischer Mengen einer organischen Phosphorverbindung der allgemeinen Formel III

$R^1R^2R^3P(X)_n$ (III),

in der

$R^1$,$R^2$,$R^3$ Wasserstoff, $C_1$- bis $C_{20}$-Alkyl, $C_6$- bis $C_{14}$-Aryloxy, $C_2$- bis $C_{20}$-Alkenyl, Halogen, Aryl oder $C_7$- bis $C_{20}$-Aralkyl bedeutet, wobei $R^1$ und $R^2$, $R^1$ und $R^3$, $R^2$ und $R^3$, $R^5$ und $R^6$ eine gesättigte oder ungesättigte $C_2$- bis $C_5$-Alkylenkette

X Sauerstoff oder Schwefel und

n 0 oder 1

bedeuten, bei Temperaturen von (-20) bis +180°C und Drücken von 0,01 bis 50 bar umsetzt.

2. Verfahren nach Anspruch 1, dadurch gekennzeichnet, daß man als organische Phosphorverbindung III ein trialkyl- oder triarylsubstituiertes Phosphinoxid oder ein Triarylphosphit verwendet.

3. Verfahren nach Anspruch 1, dadurch gekennzeichnet, daß man als organische Phosphorverbindung III Triphenylphosphit, Trioctylphosphinoxid, Tributylphosphinoxid, Triphenylphosphin, Triphenylphosphinoxid oder Hexyl-, Heptyl-, Octylphosphinoxid sowie deren Isomerengemische verwendet.

4. Verfahren nach Anspruch 1, dadurch gekennzeichnet, daß man die Umsetzung in Gegenwart von 0,001 bis 20 Mol% einer organischen Phosphorverbindung III pro Äquivalent organische Thiolgruppe verwendet.

5. Verfahren nach Anspruch 1, dadurch gekennzeichnet, daß man die Umsetzung bei einer Temperatur von 0 bis 100°C durchführt.

6. Verfahren nach Anspruch 1, dadurch gekennzeichnet, daß man in Gegenwart eines inerten Lösungsmittels arbeitet.

Europäisches Patentamt

# EUROPÄISCHER RECHERCHENBERICHT

Nummer der Anmeldung

EP 92 11 8400

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | CHEMICAL ABSTRACTS, vol. 110, 1989, Columbus, Ohio, US; abstract no. 192263x, * Zusammenfassung * & HU-A-45 973 (ESZAKMAGYARORSZAGI VEGYIMUVEK) --- | 1-6 | C07C329/02 |
| A | GB-A-2 055 827 (PPG INDUSTRIES) * Anspruch 1 * --- | 1 | |
| A | US-A-4 320 070 (J.A. COOK ET AL) * Anspruch 1 * --- | 1 | |
| D,A | US-A-3 299 114 (H. TILLES) * Anspruch 1 * ----- | 1 | |
| | | | RECHERCHIERTE SACHGEBIETE (Int. Cl.5 ) |
| | | | C07C |

Der vorliegende Recherchenbericht wurde für alle Patentansprüche erstellt

| Recherchenort | Abschlußdatum der Recherche | Prüfer |
|---|---|---|
| BERLIN | 19 JANUAR 1993 | KAPTEYN H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument